Europäisches Patentamt

European Patent Office

Office européen des brevets

(11) Veröffentlichungsnummer: **0 298 142**
**A1**

## (12) EUROPÄISCHE PATENTANMELDUNG

(21) Anmeldenummer: 87109730.9

(22) Anmeldetag: 06.07.87

(51) Int. Cl.⁴: **A61M 11/00**

(43) Veröffentlichungstag der Anmeldung:
**11.01.89 Patentblatt 89/02**

(84) Benannte Vertragsstaaten:
**AT BE CH DE ES FR GB GR IT LI LU NL SE**

(71) Anmelder: **NEDA ARZNEIMITTEL GMBH & Co.
KG
Rudolf-Diesel-Ring 21
D-8029 Sauerlach(DE)**

(72) Erfinder: **Elstner, Frank
2, rue de Sapin Senningerberg
L-1024 Luxemburg(LU)**

(74) Vertreter: **von Füner, Alexander, Dr. et al
Patentanwälte v. Füner, Ebbinghaus, Finck
Mariahilfplatz 2 & 3
D-8000 München 90(DE)**

(54) **Methode zur Aufbringung von Immunstimulantien und Umstimmungsmitteln auf Schleimhäute.**

(57) Der Gegenstand der Erfindung ist ein Verfahren zur Verabreichung von Umstimmungsmitteln oder Immunstimulantien, das dadurch gekennzeichnet ist, daß man Umstimmungsmittel-Lösungen bzw. Immunstimulans-Lösungen in an sich bekannter Weise auf die Schleimhäute des Mund- und Rachenraumes versprüht.

EP 0 298 142 A1

# METHODE ZUR AUFBRINGUNG VON IMMUNSTIMULANTIEN UND UMSTIMMUNGSMITTELN AUF SCHLEIM-HÄUTE

Die Erfindung betrifft eine Methode zur Aufbringung von Immunstimulantien und Umstimmungsmitteln auf die Schleimhäute des Mund- und Rachenraumes.

Es sind eine Reihe von Immunstimulantien und Umstimmungsmitteln bekannt, die entweder injiziert werden oder zumeist als Tabletten bzw. Tropfen zu verabreichen sind. Dabei handelt es sich um pflanzliche Reizkörper, organische und mikroorganismen-haltige Präparate sowie um Homöopathika. Beispiele sind Echinacin®, Esberitox®N, Salus®-Echinacea-Tropfen, Toxorephan®, Echinatruw®, Contramutan®, und Lymphaden®.

Diese Umstimmungsmittel oder Immunstimulantien werden u.a. zur Stärkung der Infektionsabwehr z.B. bei grippalen Infekten, gegen chronisch entzündliche Erkrankungen sowie zur Infektions-Prophylaxe eingesetzt. Injektionen können meist nur von medizinisch geschulten Personen verabreicht werden, so daß die Einnahme von Immunstimulantien auf diesem Wege nicht jedermann zugänglich ist.

Tabletten werden geschluckt, so daß die Aufnahme der Wirkstoffe dann über den Magen-Darm-Trakt vor sich geht.

Die übliche Weise ist die Einnahme von Tropfen. Dies ist jedoch umständlich, denn man muß die Tropfen abzählen, was sehr zeitaufwendig ist. Zudem benötigt man zur bequemen Einnahme auch einen Löffel oder ein kleines Glas. Die bequeme Einnahme von Tropfen-Präparaten ist also nicht überall und zu jeder Zeit möglich.

Aufgabe der Erfindung war es nun, einen Weg zu finden, durch den die Aufnahme der Umstimmungsmittel und Immunstimulantien vereinfacht und am besten auch die Wirkung vergrößert wird.

Diese Aufgabe wird wie aus den nachstehenden Ansprüchen ersichtlich gelöst.

Gegenstand der Erfindung ist also ein Verfahren zum Verabreichen von Umstimmungsmitteln sowie Immunstimulantien in Form von Lösungen, das dadurch gekennzeichnet ist, daß man die betreffenden Lösungen im Mund-Rachen-Raum in an sich bekannter Weise so versprüht, daß sich der Sprühnebel auf den Schleimhäuten des Mund- und Rachenraumes niederschlagen kann. Die verwendeten Lösungen können hierbei sowohl wässrig als auch alkoholisch sein, in Abhängigkeit von der jeweiligen Zusammensetzung.

Ein Vorteil dieser Applikationsform ist die Vereinfachung der Einnahme. Statt Tropfen abzuzählen, genügt es, das entsprechende Sprühsystem zu betätigen und so das Umstimmungsmittel oder Immunstimulans in den Mund- und Rachenraum zu applizieren. Diese Applikation kann auf bequeme Weise überall geschehen. Hilfsmittel wie Löffel oder Gläser werden nicht benötigt. Gegenüber den Tropfen-Präparaten hat die Applikationsform ferner den Vorteil, daß durch das Einsprühen der Lösung eine große Schleimhautfläche benetzt werden kann.

Bei Tropfen-Präparaten ist der Schleimhautkontakt in der Regel auf die unteren Mundpartien beschränkt. Durch die Applikation in Form eines feuchten Sprays wird dem Umstimmungsmittel oder Immunstimulans die Möglichkeit eröffnet, auf einer größtmöglichen Schleimhautfläche des Mund- und Rachenraumes zur Wirkung zu gelangen. Ein weiterer Vorteil er Applikation als feuchter Spray gegenüber den Tropfen-Präparaten ergibt sich aus der längeren Verweilzeit im Mund- und Rachenraum. Tropfen-Präparate verleiten zum Schlucken, sodaß die Verweildauer im Mund- und Rachenraum in der Regel relativ kurz ist. Durch Benetzung einer größeren Schleimhautfläche durch den feuchten Spray ist die Kontaktzeit des Immunstimulans oder Umstimmungsmittels mit der Schleimhaut verlängert, da es einige Zeit dauert, bis die feinen Tröpfchen zusamenlaufen und dann geschluckt werden.

Für das erfindungsgemäße Verfahren eignen sich alle dem Fachmann an sich bekannten Systeme, welche einen feuchten Spray erzeugen können. Ein feuchter Spray unterscheidet sich von sog. "trockenen Sprays" in der Teilchengröße des Sprühnebels, die so zu wählen ist, daß die zerteilte Lösung eine Benetzung der Schleimhaut bewirkt. Zu feine Teilchen, wie sie bei trockenen Sprays vorliegen, können zum einen in die Lungen gelangen, oder prallen von Oberflächen zurück, ohne diese zu benetzen.

Zur Erzeugung eines feuchten Sprays eignen sich Systeme, welche mit komprimierten Gasen wie $CO_2$, $N_2O$, $N_2$ oder Luft befüllt sind, sowie Systeme unter Verwendung von verflüssigten Gasen, wie Propan, Butan oder fluorierter Chlorkohlenwasserstoffe. Ebenfalls geeignet sind Systeme auf der Grundlage von Pumpzerstäubern, welche ohne zusätzliche Treibmittel auskommen. Letztere Systeme sind bevorzugt, da sie neben dem Verzicht auf Treibmittel auch eine exakte Dosierung der Umstimmungsmittel bzw. Immunstimulantien ermöglichen. Das Volumen pro Einzeldosierung und die Anzahl der Sprühstöße, welche benötigt werden, hängt, wie dem Fachmann ohne weiteres geläufig ist, von der Größe der Dosierkammer des Pumpzerstäubers und der notwendigen Tagesdosis des entsprechenden Immunstimulans bzw. Umstimmungsmittels ab. Das pro Hub zu zerstäubende Volumen wird vorzugsweise so gewählt, daß eine bequeme Anzahl von Sprühstößen genügt, um die erwünschte Dosierung zu erreichen. Das Volumen pro Hub kann zwischen 0,09 ml und 0,7 ml variieren, wobei Volumina von 0,14 bis 0,3 ml bevorzugt sind.

Die Form der Sprühdüse und des Sprühkopfes ist so auszulegen, daß ein feuchter Spray erzeugt wird, welcher eine große Schleimhautfläche des Mund- und Rachenraumes benetzen kann. Neben herkömmlichen Sprühköpfen sind auch Sonderformen, z.B. mit Rachenadaptern, geeignet.

Die Wahl des Behältermaterials hängt von dem eingesetzten Sprühsystem ab. Im Falle von komprimierten Gasen eignen sich Behälter aus Weißblech, Aluminium, Glas oder Kunststoff mit geeigneter Gasundurchlässigkeit. Im Falle von verflüssigten Treibgasen werden Behälter aus Weißblech oder Aluminium bevorzugt. Bei Verwendung der bevorzugten Pumpzerstäuber sind vom Prinzip her alle Behältermaterialien geeignet, die mit dem Füllgut kompatibel sind.

Zweckmäßigerweise werden Behälter aus Glas oder Kunststoffen eingesetzt. Das eingesetzte Behälter-Volumen richtet sich nach der zu verabreichenden Tagesdosis des Immunstimulans oder Umstimmungsmittels sowie allgemein wirtschaftlichen Überlegungen. Das auszuwählende Behältervolumen kann daher in weiten Bereichen variieren. Bevorzugt sind Volumina von 10 bis 100 ml, insbesondere solche von 30 bis 60 ml.

Die zum jeweiligen Sprühsystem zugehörigen Komponenten wie Steigrohr, Ventilteller oder Pumpenhalter werden auf den Behälter in geeigneter Weise aufgebracht. Bei Verwendung der bevorzugten Pumpzerstäuber kann dies durch Aufschrauben oder mittels Verkrimpen geschehen. Aufschraubbare Pumpzer stäuber können erforderlichenfalls auch so auf dem Behälter fixiert werden, daß ein Öffnen des Behälters nicht mehr möglich ist, und eine unerwünschte Manipulation von außen durch Dritte verhindert wird. Um ein unbeabsichtigtes Betätigen des Pumpzerstäubers zu verhindern, kann der Sprühkopf durch eine Schutzkappe oder andere geeignete Maßnahmen gesichert werden.

Beispiel 1:

Immunstimulans in flüssiger Form, bestehend aus
Echinacea purpurea Urtinktur     10,0 g
Eupatorium perfoliatum Urtinktur     10,0 g
Belladonna Urtinktur     10,0 mg ad 100,0 g mit 33 %-igem Ethanol (V/V)
abgefüllt in 50 ml Braunglas-Flaschen mit Pumpzerstäuber (0,3 ml pro Sprühstoß).
Anfangsdosis 6 Sprühstöße entsprechend 1,8 ml, danach bis zum Abklingen der Symptome stündlich 3 Sprühstöße entsprechend 0,9 ml.

Beispiel 2:

Immunstimulans in flüssiger Form, bestehend aus
Pressaft von Echinacea purpurea     60,0 g
Manit     2,0 g
Konservierungsmittel     0,01 g
Wasser     38,0 g
abgefüllt in 100 ml Kunststoff-Flaschen mit Pumpzerstäuber (0,2 ml pro Sprühstoß).
Dosierung: 3 x täglich 3-6 Sprühstöße entsprechend 0,6- 1,2 ml.

Beispiel 3:

Immunstimulans in flüssiger Form, bestehend aus
Echinacea purpurea Urtinktur     50,0 g
Eupatorium perfoliatum $D^3$     1,7 g
Thuja $D^2$     1,3 g
Aqua purificata ad     100,0 g
Die alkoholische Lösung ist in braune 30 ml-Glasflaschen abgefüllt und mit einem Dosierzerstäuber (0,2 ml Volumen pro Sprühstoß und Rachenadapter) versehen.

Anfangsdosis: 6 Sprühstöße, entsprechend 1,2 ml oder 30 Tropfen. Bis zum Abklingen der Symptome 3 x täglich 4-6 Sprühstöße, entsprechend 3 x täglich 20 - 30 Tropfen.

**Ansprüche**

1) Verfahren zum Verabreichen von Umstimmungsmitteln oder Immunstimulantien, dadurch **gekennzeichnet,** daß man Immunstimulans-Lösungen bzw. Umstimmungsmittel-Lösungen in an sich bekannter Weise auf die Schleimhäute des Mund- und Rachenraumes versprüht.

2) Verfahren nach Anspruch 1, dadurch **gekennzeichnet,** daß man an sich bekannte Pump- und Dosierzerstäuber verwendet.

3) Verfahren nach Anspruch 2, dadurch **gekennzeichnet,** daß man pro Sprühstoß 0,09 bis 0,7 ml aufträgt.

4) Verfahren nach Anspruch 3, dadurch **gekennzeichnet,** daß man pro Sprühstoß 0,15 bis 0,3 ml aufträgt.

| Europäisches Patentamt | **ERKLÄRUNG,** die nach Regel 45 des Europäischen Patent- übereinkommens für das weitere Verfahren als europäischer Recherchenbericht gilt | Nummer der Anmeldung EP  87 10 9730 |

| Nach Auffassung der Recherchenabteilung entspricht die vorliegende europäische Patentanmel- dung den Vorschriften des Europäischen Patentübereinkommens so wenig, daß es nicht möglich ist, auf der Grundlage aller Patentansprüche sinnvolle Ermittlungen über den Stand der Technik durchzuführen. Grund: | KLASSIFIKATION DER ANMELDUNG (Int. Cl.4) |
|---|---|
| Verfahren zur chirurgischen oder therapeutischen Behandlung des menschlichen oder tierischen Körpers (Siehe Art. 52(4) des Europäischen Patentübereinkommens). | A 61 M 11/00 |

| Recherchenort Den Haag | Abschlußdatum der Recherche 20-08-1987 | Prüfer VEREECKE |

EPA Form 1504   06.78